# EUROPEAN PATENT SPECIFICATION

(11) **EP 1 357 819 B1**
(45) Date of publication and mention of the grant of the patent: **10.10.2007**
(21) Application number: 02709262.6
(22) Date of filing: 01.02.2002
(51) Int. Cl.: A45D 44/00, A61K 8/02, A61K 8/29, A61K 8/73, A61K 8/67, A61Q 1/02, A61Q 19/02

(54) **MASK COMPOSITION**
GESICHTSMASKE
MASQUE COSMETIQUE

(30) Priority: 08.02.2001 WO PCT/US01/04069
(43) Date of publication of application: 05.11.2003
(73) Proprietor: THE PROCTER & GAMBLE COMPANY, Cincinnati, Ohio 45202 (US)
(72) Inventor: TRIGG, David Leigh, Hyogo-Ken, 665-0885 (JP); CHEN, Yin-Jang, Kobe, Hyogo 654-0053 (JP); CHEN, Minghua, Kobe, Hyogo 520-3241 (JP)
(74) Representative: Wilding, Richard Alan
(86) International application number: PCT/US2002/002859
(87) International publication number: WO 2002/062132

(56) References cited:
- EP-A2- 0 063 875
- WO-A1-00/28961
- WO-A2-02/062132
- DE-A1- 10 017 530
- US-B1- 6 337 066

## Description

### FIELD

The present invention relates to a mask composition for topical application which contains a skin tone changing agent for providing acute skin tone changing benefit.

### BACKGROUND

Masks designed for providing treatment to the skin are known in the art, such as SKII Facial Treatment Mask on the Japanese market. Such masks are made of a substrate and a liquid soaked in the substrate, wherein the mask is adhered only very weakly to the skin, such that the mask is easily removed from the skin with practically no tension to the skin. These treatment masks can be distinguished from removal masks. Removal masks are those designed to firmly adhere to the skin and thereby remove dirt, clogs, and excess corneum on the surface and in the pores of skin upon peeling off the mask. Treatment masks are particularly suitable for applying to the skin for delivering moisturizing agents and other benefit agents to the skin through a wet, typically aqueous, environment. In fact, delivery of moisturizing agents and other benefit agents via a mask is advantageous in that the skin is exposed to an abundant amount of such agents over a lengthy period of time. Treatment masks also provide relaxation benefit to the user upon use, because the usage encourages the user to sit or lay down. Treatment masks are generally applied to the facial skin.

Consumers frequently use cosmetic products to care for their skin as well as to improve the health and/or physical appearance of their skin. Rough and/or broken skin and hyperpigmentations (such as age spots, freckles, and brown patches associated with sunlight exposure, skin aging or environmental damage to the human skin) are areas consumers typically seek to treat. Skin whitening is of particular interest in certain Asian populations.

A wide variety of compounds and/or ingredients, *e.g.*, ascorbic acid and derivatives thereof, kojic acid and derivatives thereof, hydroquinone, arbutin, and a variety of extracts such as glycyrrhiza, are known and are commonly-available for skin-whitening use. While such compounds can be incorporated in a mask, the effect of such compounds for whitening the skin is typically seen after a chronic and repetitive use of the mask. It would therefore be desirable to provide an acute whitening benefit by the use of a treatment mask. Further, for consumers who wish to alter their skin to a darker, brighter, or to a different color and/or tone, it would be desirable to provide a treatment mask providing such benefits. Overall, such needs can be described as a need to change the skin tone.

Based on the foregoing, there is a need for a mask composition which provides acute skin tone changing benefit to the skin, and particularly, a mask which can effectively deliver such benefits via a stable mask composition. There is further a need for a mask composition which provides both acute and chronic whitening benefit to the skin.

None of the existing art provides all of the advantages and benefits of the present invention.

### SUMMARY

The present invention is directed to a mask composition comprising:
(1) a water insoluble non-woven substrate; and
(2) a liquid composition comprising:
   (a) a skin tone changing material selected from the group consisting of skin tone changing pigments, reflective particulate material, and mixtures thereof, wherein the skin tone changing agent has a particle size of at least about 100nm;
   (b) a water-soluble thickening agent which provides the liquid composition a viscosity of from about 1000 mPa s to about 600,000 mPa s; and
   (c) an aqueous carrier.

These mask compositions provide a stable composition which provides acute skin tone changing benefit for the skin upon use.

These and other features, aspects, and advantages of the present invention will become better understood from a reading of the following description, and appended claims.

### BRIEF DESCRIPTION OF THE FIGURE

While the specification concludes with claims particularly pointing out and distinctly claiming the invention, it is believed that the present invention will be better understood from the following description of preferred, nonlimiting embodiments and representations taken in conjunction with the accompanying drawings in which:
Fig. 1 is a plane view of a preferred embodiment of the water-insoluble substrate of the present invention.
Fig. 2 is an inflated cross sectional view of the water-insoluble substrate having an occluded side.

### DETAILED DESCRIPTION

While the specification concludes with claims particularly pointing out and distinctly claiming the invention, it is believed that the present invention will be better understood from the following description.

Citation of any reference is not an admission regarding any determination as to its availability as prior art to the claimed invention.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "topical application" means to apply or spread a material onto the surface of the skin.

Herein, "skin tone changing" refers altering the appearance of the color and/or tone of the skin including, but not limited to, skin whitening.

Herein, "skin whitening" refers altering the appearance of the skin to a brighter, lighter, and/or whiter appearance.

Herein, "cosmetically-acceptable" means that the compositions or components thereof so described are suitable for use in contact with human skin without undue toxicity, incompatibility, instability, allergic response, and the like.

Herein, "safe and effective amount," means an amount of a compound or composition sufficient to significantly induce a positive benefit, preferably a positive skin appearance or feel benefit, including independently the benefits disclosed herein, but low enough to avoid serious side effects, e.g., to provide a reasonable benefit to risk ratio, within the scope of sound judgment of the skilled artisan.

Herein, "mixtures" is meant to include a simple combination of materials and any compounds that may result from their combination.

All ingredients such as actives and other ingredients useful herein may be categorized or described by their cosmetic and/or therapeutic benefit or their postulated mode of action. However, it is to be understood that the active and other ingredients useful herein can, in some instances, provide more than one cosmetic and/or therapeutic benefit or operate via more than one mode of action. Therefore, classifications herein are made for the sake of convenience and are not intended to limit an ingredient to the particularly stated application or applications listed.

### WATER-INSOLUBLE SUBSTRATE

The mask compositions of the present invention comprise a water-insoluble non-woven substrate. By "water insoluble", it is meant that the substrate does not dissolve in or readily break apart upon immersion in water. The water-insoluble substrate is the implement or vehicle for delivering the liquid composition to the skin.

A wide variety of materials can be used as the substrate. The following nonlimiting characteristics are desirable: (i) sufficient wet strength for use, (ii) sufficient abrasivity, (iii) sufficient thickness, (iv) appropriate size, (v) air permeability, and (vi) hydrophobicity.

Nonwoven substrates are economical and readily available in a variety of materials. By "nonwoven", it is meant that the layer is comprised of fibers which are not woven into a fabric but rather are formed into a sheet, mat, or pad layer. The fibers can either be random (i.e., randomly aligned) or they can be carded (i.e., combed to be oriented in primarily one direction). Furthermore, the nonwoven substrate can be composed of a combination of layers of random and carded fibers:

Nonwoven substrates may be comprised of a variety of materials both natural and synthetic. By "natural", it is meant that the materials are derived from plants, animals, insects or byproducts of plants, animals, and insects. By "synthetic", it is meant that the materials are obtained primarily from various man-made materials or from natural materials which have been further altered. The conventional base starting material is usually a fibrous web comprising any of the common synthetic or natural textile-length fibers, or mixtures thereof.

Nonlimiting examples of natural materials useful in the present invention are silk fibers, keratin fibers and cellulosic fibers. Nonlimiting examples of keratin fibers include those selected from the group consisting of wool fibers, camel hair fibers, and the like. Nonlimiting examples of cellulosic fibers include those selected from the group consisting of wood pulp fibers, cotton fibers, hemp fibers, jute fibers, flax fibers, and mixtures thereof.

Nonlimiting examples of synthetic materials useful in the present invention include those selected from the group consisting of acetate fibers, acrylic fibers, cellulose ester fibers, modacrylic fibers, polyamide fibers, polyester fibers, polyolefin fibers, polyvinyl alcohol fibers, rayon fibers, polyurethane foam, and mixtures thereof. Examples of some of these synthetic materials include acrylics such as acrilan, creslan, and the acrylonitrile-based fiber, orlon; cellulose ester fibers such as cellulose acetate, amel, and acele; polyamides such as nylons (e.g., nylon 6, nylon 66, nylon 610, and the like); polyesters such as fortrel, kodel, and the polyethylene terephthalate fiber, dacron; polyolefins such as polypropylene, polyethylene; polyvinyl acetate fibers; polyurethane foams and mixtures thereof. These and other suitable fibers and the nonwoven materials prepared therefrom are generally described in Riedel, "Nonwoven Bonding Methods and Materials," Nonwoven World (1987); The Encyclopedia Americana, vol. 11, pp. 147-153, and vol. 26, pp. 566-581 (1984); U.S. Patent No. 4,891,227, to Thaman et al., issued January 2, 1990; and U.S. Patent No. 4,891,228.

Nonwoven substrates made from natural materials consist of webs or sheets commonly formed on a fine wire screen from a liquid suspension of the fibers. See C.A. Hampel et al., The Encyclopedia of Chemistry, third edition, 1973, pp. 793-795 (1973); The Encyclopedia Americana, vol. 21, pp. 376-383 (1984); and G.A. Smook, Handbook of Pulp and Paper Technologies, Technical Association for the Pulp and Paper Industry (1986).

Substrates made from natural materials useful in the present invention can be obtained from a wide variety of commercial sources. Nonlimiting examples of suitable commercially available paper layers useful herein include AIRTEX^{®}, an embossed airlaid cellulosic layer having a base weight of about 71 gsy (grams per square yard), i.e., about 85 gsm (grams per square meter), available from James River, Green Bay, WI; and WALKISOFT^{®}, an embossed airlaid cellulosic having a base weight of about 75 gsy (about 90 gsm), available from Walkisoft U.S.A., Mount Holly, NC.

Nonwoven substrates made from synthetic materials useful in the present invention can also be obtained from a wide variety of commercial sources. Nonlimiting examples of suitable nonwoven layer materials useful herein include NOVONET^{®} 149-616, a thermo-bonded grid patterned material containing about 100% polypropylene, and having a basis weight of about 50 gsy (about 60 gsm), available from Veratec, Inc., Walpole, MA; NOVONET^{®} 149-801, a thermo-bonded grid patterned material containing about 69% rayon, about 25% polypropylene, and about 6% cotton, and having a basis weight of about 75 gsy (about 90 gsm), available from Veratec, Inc. Walpole, MA; NOVONET^{®} 149-191, a thermo-bonded grid patterned material containing about 69% rayon, about 25% polypropylene, and about 6% cotton, and having a basis weight of about 100 gsy (about 120 gsm), available from Veratec, Inc. Walpole, MA; KEYBAK^{®} 951V, a dry formed apertured material, containing about 75% rayon, about 25% acrylic fibers, and having a basis weight of about 43 gsy (about 51 gsm), available from PGI/Chicopee, Dayton, NJ; KEYBAK^{®} 1368, an apertured material, containing about 75% rayon, about 25% polyester, and having a basis weight of about 39 gsy (about 47 gsm), available from PGI/Chicopee, Dayton, NJ, RMT-90, a 3-layer substrate having a pulp layer as an inner layer with outer layers respectively made of the combination of rayon and polyester, and RFP-90, a 3-layer substrate having a combined PET/PE layer as an inner layer with outer layers of rayon and polyester, both available from Daiwabo K.K.

In the present invention the nonwoven layer can be prepared by a variety of processes including hydroentanglement, thermally bonding or thermo-bonding, and combinations of these processes. Methods of making nonwoven substrates are well known in the art. Generally, these nonwoven substrates can be made by air-laying, water-laying, meltblowing, coforming, spunbonding, or carding processes in which the fibers or filaments are first cut to desired lengths from long strands, passed into a water or air stream, and then deposited onto a screen through which the fiber-laden air or water is passed.

Processes for preparing hydroentangled webs are well known in the art. See, for example, Evans; U.S. Patent 3,485,786; issued December 23, 1969; Kalwarres; U.S. Patent 2,862,251 and Griswold; U.S. Patent 3,025,585, all of which describe hydroentangling procedures generally. See also U.S. Patent 5,674,591; James et al; issued October 7, 1997 which specifically describes a hydroentangling process, including the apparatus used in said process, which can be used to prepare the patterned web.

Among the water-insoluble substrate described hereinbefore, fluid-entangled (hydroentangled), nonwoven, flexible substrates are preferably used in the present invention, in view of providing softness, loftness, strength while being used, and cloth-like texture. Any fluid-entangled, nonwoven, flexible substrate that is known or is otherwise suitable for application to the skin can be used in the mask compositions of the present invention.

The term "fluid-entangled" as used herein is an art recognized term which refers generally to the manufacturing process for entangling a fibrous web by using a fluid jet on a fibrous web to obtain the desired fiber and void configuration within the resulting fluid-entangled substrate, to thereby produce an art recognized, fluid-entangled, nonwoven, flexible substrate. Fluid-entangled, nonwoven, flexible substrates and the fluid entangling techniques for making them are well known in the substrate arts, preferred examples of such substrates and fluid entangling techniques being described in U.S. Patents 5,142,752 (Greenway et al.) and U.S. Patents 5,281,461 (Greenway et al.).

These preferred fluid-entangled, nonwoven, flexible substrates comprise a symmetrical array of entangled staple fibers including a lattice structure of spaced parallel machine direction oriented rows of criss-crossing fibrous bands, and spaced cross-direction oriented fibrous bands, said machine direction fibrous bands and said cross-direction fibrous bands intersecting at dense fiber nodes, said symmetrical array of fibers having a ratio in the range of 1/1 to 4/1; wherein said symmetrical array of fibers is fluid entangled by discrete streams of focused fluid energy which have a confluence corresponding to said symmetrical array. More preferably, the machine direction fibrous bands and the criss-crossing fibrous bands further comprise connecting interstitial fiber components which substantially occupy interstitial spaces defined by said fibrous bands, such that the fabric has a non-apertured textile-like finish.

These substrates are preferably a blend of polyester and rayon or cotton fibers and have a weight in the range of 40-120 gsy (grams per square yard), i.e., 48-144 gsm (grams per square meter). These preferred substrates are typically prepared by using an apparatus and related process for entangling a fibrous web which employs columnar fluid jets to eject a continuous curtain of fluid in an entangling station. The web is advanced through an entangling station on a conveyor which supports an entangling member having a symmetrical pattern of void areas. Baffle members disposed in the void areas are provided which include radiused curvatures and define apertures having a frusto-conical configuration. Dynamic forces in the fluid curtain impact the web in discrete and controlled patterns determined by the baffling members to enhance efficient energy transfer and web entanglement. Textile-like fabrics having a uniform, non-apertured, surface cover are obtained by coaction of fluid curtain and baffle structures.

Other known techniques for making fluid-entangled, nonwoven, flexible substrates are described, for example in U.S. Patent 3,485,786 (Evans); U.S. Patent 2,862,251 (Kalwarres); and U.S. Patent 3,025,585 (Griswald).

Other suitable methods of making fluid-entangled substrates are described in U.S. Patent 5,674,591 (James et al.) which specifically describes a hydroentangling process, including the apparatus used in said process, which can be used to prepare a patterned web.

The resulting layer, regardless of its method of production or composition, is then subjected to at least one of several types of bonding operations to anchor the individual fibers together to form a self-sustaining web. Moreover, the substrates of the present invention can consist of a single layer or multiple layers. In addition, multilayered substrates can include films and other nonfibrous materials. In one embodiment, the substrate may also be laminated with polymeric film on the substrate, coating the substrate, or heat sealing the substrate. The resulting substrate with the laminated polymeric film, coating or heat sealing comprises an occluded side on one side of the substrate, which faces away from the skin, and a skin facing side that is positioned on the skin surface. By having a substrate with an occluded side, the substrate acquires low air permeability. By "low air permeability" it is meant that the side of the substrate having the film, coating or heat sealing allows very little air to enter into the substrate and very little vapor to escape from the substrate. Preferably the air permeability is less than about 5 mg/cm²/min, more preferably between about 0.01 mg/cm²/min and about 4.8 mg/cm²/min. The air permeability can be measured by taking the weight of a fully saturated sample of the substrate and weighing the substrate after it is exposed to the atmosphere.

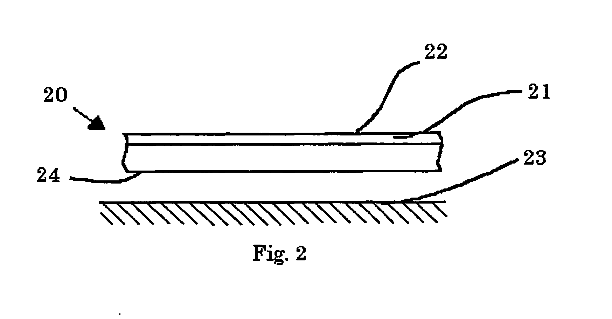

An embodiment of a substrate having an occluded side having low air permeability is shown in Figure 2. As shown in Figure 2, a substrate (20) is laminated with a polymer film (21), forming an occluded side (22). The polymer film (21) is greatly exaggerated to show detail. The occluded side (22), is placed away from the skin surface (23) during use. The occluded side (22) restricts moisture from escaping into the atmosphere during a typical use time period between about 5 minutes to about 45 minutes thus providing improved moisturizing effect to the skin. The skin facing side (24) of the substrate (20) preferably does not contain any materials that restrict air permeability.

A substrate comprising an occluded side significantly increases penetration of chronic whitening agents and skin benefit agents into the skin compared to a substrate without an occluded side. Without being limited by a theory, it is believed that the occluded side (22) of the substrate (20) allows for the creation of a humid environment near the surface of the skin by limiting the evaporation of water from the mask composition into the atmosphere. Additionally, the mask composition of the present invention utilizes water tension to adhere to the skin surface rather than strong adhesives on the skin facing side. The absence of a strong adhesive between the substrate and the skin surface, as utilized by the present invention, removes the physical barrier resulting from the strong adhesive and promotes the penetration of the chronic whitening agents and the skin benefits agents. The resulting environment between the skin and the substrate promotes the penetration of the chronic skin whitening agents and the skin benefit agents into the skin.

Suitable polymeric film includes polyethylene, polypropylene, polyethylene terephtalate, polyamides, polyesters, nylons, blends thereof, or any other cosmetically acceptable polymeric films. Suitable coatings include any materials know in the art that impart low air permeability to the substrate and are cosmetically acceptable. Heat-sealing the substrate may be accomplished by any method known in the art to impart low air permeability to the substrate.

Substrate materials particularly useful herein include those that are of hydrophilic nature, thereby capable of absorbing a larger quantity of the liquid composition. The water-insoluble substrate can be made solely of hydrophilic material, or made of layers of substrates wherein at least the layer facing the skin is made of hydrophilic material.

The substrate may also be made by a combination of hydrophilic material and hydrophobic material. In one preferred embodiment, the substrate is made of at least partially by hydrophilic materials selected from cotton, pulp, rayon, and mixtures thereof. By partially, it is meant to encompass both situations where at least a layer of a hydrophilic substrate is used, and where at least some of the hydrophilic material is used in combination with another material to make a mixed substrate. In one embodiment the mixed substrate comprises materials having increasing hydrophobic properties from the skin facing side to the occluded side. Referring to Figure 2, the skin facing side of the substrate faces the skin and comprises hydrophilic material. Moving through the cross section of the substrate to the occluded side that is in contact with the ambient air, the substrate utilizes more hydrophobic materials. By "hydrophobic" it is meant that the materials in this portion of the substrate have a contact angle with moisture greater than about 10 degrees, preferably between about 10 degrees and 90 degrees as measured by ASTM D 5725-99. This structure allows for moisture to be move from the hydrophobic occluded side of the substrate to the hydrophilic skin facing side of the substrate to the skin surface. These preferred structure could be accomplished by having a single layer or a laminated structure.

The substrate can be made into a wide variety of shapes and forms such as flat pads, thick pads, thin sheets, and sheets of irregular thickness, depending on the desired use and characteristic of the mask. The substrate is typically designed to fit the area of the skin to which topical application is desired. For example, when the mask is applied to the face, the substrate is designed to correspond to the shape of the face avoiding the eye, nostril, and mouth areas, as necessary.

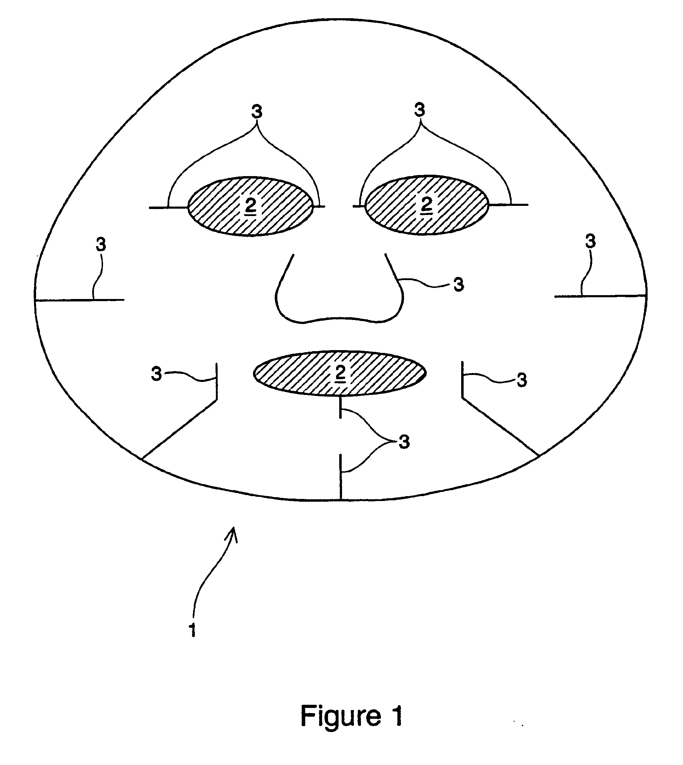

In one preferred embodiment, the substrate is so configured to cover substantially the whole area of the facial skin with areas of the eyes and nostrils opened. Referring to Figure 1, a plane view of a particularly preferred embodiment of a substrate suitable for a single-piece whole facial mask (10) is depicted. The outer peripheral of the substrate of Figure 1 is designed to approximately match the contour of the face, with a plurality of openings (12) for the eyes and the mouth, and wherein a plurality of cuttings (13) are made so that the mask fits the nose, cheeks, and the mouth. The embodiment of Figure 1 has a length of from about 15cm to about 25cm, preferably from about 18cm to about 23cm, and a width of from about 15cm to about 30cm, preferably from about 20cm to about 25cm; to cover the average entire facial area. In another preferred embodiment, the substrate is so configured to cover substantially the whole area of the facial skin, and is made of two pieces, the first piece covering the upper area of the face, i.e. the nose and thereabove, and the second piece covering the lower area of the face, i.e. the upper lip, cheeks and thereunder. In another preferred embodiment, the substrate is so configured to match the area of a particular part of the face, such as the nose, cheekbone, chin, forehead, or combinations thereof.

The substrate is flexible enough such that, when soaked with the liquid composition, readily fits along the skin, yet strong enough so that it does not easily tear or crumble upon use. Preferably, the substrate has a thickness of from about 100 µm to about 1 cm, more preferably from about 300 µm to about 3 mm, depending on the material for making the substrate, and use and characteristic of the product. In one embodiment, a cotton substrate having a relatively thick thickness of about 2mm is preferred for providing a natural, lofty, cloth-like appearance, and for soaking an abundant amount of the liquid composition. In another embodiment, a substrate made of layers of substrates, wherein the layer to be applied to the face is made of hydrophilic material, and having a relatively thin thickness of about 0.5mm, is preferred for providing a fresh, soft appearance, and for soaking just about the amount of liquid composition necessary for depositing the skin tone changing agent.

### LIQUID COMPOSITION

The mask compositions of the present invention comprise a liquid composition in addition to the water-insoluble substrate described hereinbefore. The mask composition of the present invention comprises a liquid composition that impregnates, coats or is otherwise in contact with the water-insoluble substrate described hereinbefore.

The liquid composition herein comprises:
(a) a skin tone changing agent selected from the group consisting of skin tone changing pigments, reflective particulate material, and mixtures thereof, wherein the skin tone changing material has a particle size of at least about 100nm;
(b) a water-soluble thickening agent which provides the liquid composition a viscosity of from about 1000 mPa s to about 600,000 mPa s; and
(c) an aqueous carrier;

The amount of liquid composition associated with any individual mask composition will vary depending upon the desired characteristics of the finished mask composition product, but should be at least an amount sufficient to result in deposition of the skin tone changing agent onto the skin during application to provide a noticeable acute skin tone changing benefit. The mask compositions of the present invention are preferably saturated with the liquid composition such that the water-insoluble substrate remains saturated with the liquid composition. To that desired end, the liquid composition will therefore preferably represent from about 100% to about 2000%, more preferably from about 200% to about 1500%, by weight of the water-insoluble substrate. By the phrase "remains saturated with the liquid composition," as used herein means that the substrate is not dried during the interval between the saturation of the mask composition with the liquid composition and application of the mask composition to the skin. The amount of liquid composition to be used will depend on the absorbing capability of the water-insoluble substrate, and the desired characteristic of the mask composition.

The liquid compositions used for the mask composition of the present invention have a viscosity in the range of from about 1000 mPa s to about 600,000 mPa s, preferably from about 2000 mPa s to about 300,000 mPa s, more preferably from about 3000 mPa· s to about 100,000 mPa s, as measured by a Brookfield Digital Viscometer, Model DV-II+ Version 3.2 according to the operating instructions set forth in Manual No. M/92-161-H895. Such viscosity is believed to be suitable for suspending the skin tone changing agents in the liquid composition in an effective manner, as well as effectively depositing the skin tone changing agents and other benefit agents to the skin.

### SKIN TONE CHANGING AGENT

The liquid compositions of the present invention comprise a skin tone changing agent selected from the group consisting of skin tone changing pigments, reflective particulate material, and mixtures thereof, wherein the skin tone changing agent has a particle size of at least about 100nm.

The skin tone changing agents useful herein can be inorganic or organic, so long as they are insoluble and non-reactive with the liquid composition, and have a suitable density such that they remain dispersed in the liquid composition in a stable manner. The skin tone changing agents, when applied to the skin via topical application of the mask, provide an acute skin tone changing benefit. By definition, "acute" herein means an effect provided by 1 to 5 times usage, preferably a 1-time usage. The skin tone changing agents herein are included, by weight of the liquid composition, at a level sufficient to provide the acute skin tone changing benefit, preferably from about 0.001% to about 5%, more preferably from about 0.01% to about 2%, still preferably from about 0.1% to about 2%.

### Reflective Particulate Material

The reflective particulate materials useful in the compositions of the present invention are those which provide a noticeable whitening effect on the skin when applied. Reflective particular materials particularly useful herein will have a refractive index of at least about 2, more preferably at least about 2.5. Refractive index can be determined by conventional methods. For example, a method for determining the refractive index which is applicable to the present invention is described in J. A. Dean, Ed., Lange's Handbook of Chemistry, 14th Ed., McGraw Hill, New York, 1992, Section 9, Refractometry.

The reflective particulate materials herein have a primary particle size of from about 100nm to about 10µm (i.e., in the essentially pure, powder form prior to combination with any carrier). The particle size is selected depending on the characteristic of the reflective particulate material. Primary particle size can be determined using the ASTM Designation E20 - 85 "Standard Practice for Particle Size Analysis of Particulate Substances in the Range of 0.2 to 75 Micrometers by Optical Microscopy", ASTM Volume 14.02, 1993.

### (i) Inorganic Reflective Particulate Material

The inorganic reflective particulate materials useful herein include metal oxides such as titanium dioxide, zinc oxide, zirconium dioxide, aluminum oxide, and combinations thereof, more preferably titanium dioxide, zinc oxide and combinations thereof (combinations are intended to include particles which comprise one or more of these materials, as well as mixtures of these particulate materials) and most preferably, the particles consist essentially of titanium dioxide. The inorganic reflective particulate material may be a composite, e.g., deposited on a core or mixed with other materials such as, but not limited to, silica, silicone resin, mica, and nylon. Preferably the inorganic reflective particular materials have a primary particle size when dispersed in the composition of from about 100 nm to about 1000 nm, more preferably from about 100 nm to about 400 nm even more preferably from about 200 nm to about 300 nm.

Inorganic reflective particulate materials, e.g., titanium dioxide, zinc oxide, zirconium dioxide, or aluminum oxide are commercially available from a number of sources. One example of a suitable particulate material comprises TRONOX™ (titanium dioxide series) and SAT-T CR837 (a rutile Titanium dioxide) available from U. S. Cosmetics, titanium dioxide CR-50 available from Ishihara Sangyo Kaisha, and titanium dioxide JA-1 available from Tayca Corporation.

The metal oxide materials herein are preferably coated with a coating material that confers a net charge that is greater than the zeta potential of the uncoated metal oxide. Such charging is advantageous for dispersing the reflective particulate material throughout the liquid composition.

Without being limited by theory, it is believed that reflective particulate materials, such as titanium dioxide, generally possess relatively high surface activity, creating formulation instabilities. In addition, these ,reflective particulate materials have a tendency to agglomerate, e.g., clump together, resulting in precipitation of the reflective particulate materials. These problems can be solved by coating the reflective particulate material (metallic oxide) with a coating material that confers a net charge that is greater than the zeta potential of the uncoated reflective particulate material. Typically, the coating material confers a zeta potential that is greater than about ±20 mV (e.g., either in the positive or negative direction) at pH from about 4 to about 8.5. Further such coating materials provide reflective particulate materials with steric hindrance, resulting in preventing agglomeration of such charged materials. This provides formulation stability and prevents agglomeration of the reflective particulate materials (metallic oxide). Particulates and their charges are well known to those of ordinary skill in the art, and are well described in R.J Hunter, Zeta Potential in Colloid Science: Principles and Application (1981), published by Academic Press; J.N. Israelachvili, Intermolecular and Surface Forces: With Applications to Colloidal and Biological Systems (1985), published by Academic Press; and Hoogeven, N.G. et al., Colloids and Surfaces, Physiochemical and Engineering Aspects, Vol. 117, p. 77 (1966).

Preferably, the charged materials all have a net cationic charge or a net anionic charge. It is believed that because all of the particles have the same charge, the repulsive forces prevent agglomeration and induce even distribution throughout the hydrophilic phase. As a result, (i) lower concentrations of the reflective particulate material give maximum visible light reflection in a composition, (ii) aesthetic negative impact such as chalky and gritty feel are not created, and (iii) formulation instabilities are decreased. Thus, the use of charged materials provide efficient coverage at relatively low levels of the charged materials.

The particles may have a variety of shapes, including spherical, spheroidal, elliptical, lamellar, irregular, needle and rod-like, provided that the desired refractive index is provided. The particulate can be in a variety of physical forms, including rutile, anatase or a combination thereof.

### (ii) Coating Material

The inorganic reflective particulate materials described hereinbefore are preferably coated with a coating material that confers a net charge that is greater than the zeta potential of the uncoated reflective particulate material. Therefore, any coating material can be used as long as the net charge (cationic or anionic) conferred to the reflective particulate is greater than the untreated reflective particulate material. However, all of the particulates within a composition are preferably treated with the same net charge, *e.g.*, no mixing of cationic and anionic coating materials, to benefit from the repulsive forces between the reflective particulates. It is understood by one skilled in the art, however, that small amounts of oppositely charged coating materials may be used, as long as, the overall repulsive forces are maintained.

Nonlimiting examples of coating materials that confer a cationic charge include cationic polymers (natural and/or synthetic) and cationic surfactants. Preferred cationic coating materials are selected from the group consisting of chitosan, hydroxypropyl chitosan, quaternium-80, polyquaternium-7, and mixtures thereof.

Nonlimiting examples of coating materials that confer an anionic charge include anionic polymers (natural and/or synthetic) and anionic surfactants. Preferred anionic coating materials are selected from the group consisting of ammonium polyacrylate, sodium polyacrylate, potassium polyacrylate, ethylene acrylic acid copolymer, hydrolyzed wheat protein polysiloxane copolymer, dimethicone copolyol phosphate, dimethicone copolyol acetate, dimethicone copolyol laurate, dimethicone copolyol stearate, dimethicone copolyol behenate, dimethicone copolyol isostearate, dimethicone copolyol hydroxystearate, phosphate ester, sodium chondroiton sulfate, sodium hyaluronate, ammonium hyaluronate, sodium algenate, ammonium algenate, ammonium laurate, sodium laurate, potassium laurate, ammonium myristate, sodium myristate, potassium myristate, ammonium palmitate, sodium palmitate, potassium palmitate, ammonium stearate, sodium stearate, potassium stearate, ammonium oleate, sodium oleate, potassium oleate, and mixtures thereof. More preferred are anionic coating materials selected from the group consisting of ammonium polyacrylate, sodium polyacrylate, and mixtures thereof.

The charged materials (*e.g.,* treated with the coating material) are available in essentially neat, powdered form, or predispersed in various types of carriers, including but not limited to water, organic hydrophilic diluents such as lower monovalent alcohols (*e.g*., C₁₋₄) and low molecular weight glycols and polyols, including propylene glycol, polyethylene glycol (*e.g.*, Molecular Weight 200-600 g/mole), polypropylene glycol (e.g., Molecular Weight 425-2025 g/mole), glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof. Preferably, the charged particulate materials are predispersed in water, glycerin, butylene glycol, propylene glycol, and mixtures thereof. Examples of commercially available charged particulate materials include Kobo BG60DC (a predispersion of chitosan treated titanium dioxide and butylene glycol), Kobo GLW75CAP (a predispersion of ammonium polyacrylate treated titanium dioxide, water, and glycerin), Kobo GLW75CAP-MP (a predispersion of ammonium polyacrylate treated titanium dioxide, water, glycerin, methylparaben, and propylparaben) all available from Kobo Products Inc., located in South Plainfield, NJ.

### (iii) Organic Reflective Particulate Material

The organic reflective particulate materials useful herein include various grades of starch which meet the needs as described hereinabove.

Particularly useful starch material herein include those known as "resistant starch" in the foods field. Surprisingly, it has been found that these resistant starches are advantageous for the mask composition of the present invention. Resistant starch is the starch fraction not digested in the small intestine of healthy human individuals, due to inaccessibility of the starch to digestive enzymes caused by crystallization, complexation or physical isolation. Such resistant starch have less water absorbency than regular edible starches, and therefore provide a higher refractive index as required for the reflective particulate materials herein. Further, resistant starch has less tendency to increase the viscosity of the liquid composition to an unacceptable degree, and less tendency of providing unacceptable negative sticky, tacky feeling to the skin when applied.

Resistant starches particularly useful herein include commercially available materials such as CrystaLean® available from Opta Food Ingredients Inc., Novelose 240® available from National Starch and Chemical Company Inc., Amylomaize® VII available from Cerestar USA Inc., and Hylon® VII available from National Starch and Chemical Company Inc.

### Skin Tone Changing Pigments

The skin tone changing pigments useful herein are those which have a particle size of at least about 100 nm. Exemplary skin tone changing pigments herein include clay mineral powders such as talc, mica, sericite, silica, magnesium silicate, synthetic fluorphlogopite, calcium silicate, aluminum silicate, bentonite and montomorilonite; pearl pigments such as alumina, barium sulfate, calcium secondary phosphate, calcium carbonate, titanium oxide, finely divided titanium oxide, zirconium oxide, zinc oxide, hydroxy apatite, iron oxide, iron titate, ultramarine blue, Prussian blue, chromium oxide, chromium hydroxide, cobalt oxide, cobalt titanate, titanium oxide coated mica; organic powders such as polyester, polyethylene, polystyrene, methyl metharylate resin, cellulose, 12-nylon, 6-nylon, styrene-acrylic acid copolymers, poly proprylene, vinyl chloride polymer, tetrafluoroethylene polymer, boron nitride, fish scale guanine, laked tar color dyes, and laked natural color dyes.

### WATER SOLUBLE THICKENING AGENT

The liquid compositions of the present invention comprise a water-soluble thickening agent. The water soluble thickening agents herein are water soluble or water miscible polymers, have the ability to increase the viscosity of the composition, and are compatible with the skin tone changing agent. The water-soluble thickening agent is selected so that the liquid composition of the present composition has the desired viscosity of from about 1000 mPa s to about 600,000 mPa s, preferably from about 2000 mPa s to about 300,000 mPa s, more preferably from about 3000 mPa s to about 100,000 mPa s. The water soluble thickening agents are included, by weight of the liquid composition, at a level preferably from about 0.1% to about 3%, more preferably from about 0.1% to about 2%, still preferably from about 0.2% to about 2%.

Water soluble thickening agents useful herein include anionic polymers and nonionic polymers. Useful herein are vinyl polymers such as cross linked acrylic acid polymers with the CTFA name Carbomer, cellulose derivatives and modified cellulose polymers such as methyl cellulose, ethyl cellulose, hydroxyethyl cellulose, hydroxypropyl methyl cellulose, nitro cellulose, sodium cellulose sulfate, sodium carboxymethyl cellulose, crystalline cellulose, cellulose powder, polyvinylpyrrolidone, polyvinyl alcohol, guar gum, hydroxypropyl guar gum, xanthan gum, arabia gum, tragacanth, galactan, carob gum, guar gum, karaya gum, carragheenin, pectin, agar, quince seed (Cydonia oblonga Mill), starch (rice, com, potato, wheat), algae colloids (algae extract), microbiological polymers such as dextran, succinoglucan, pulleran, starch-based polymers such as carboxymethyl starch, methylhydroxypropyl starch, alginic acid-based polymers such as sodium alginate, alginic acid propylene glycol esters, acrylate polymers such as sodium polyacrylate, polyethylacrylate, polyacrylamide, polyethyleneimine, and inorganic water soluble material such as bentonite, aluminum magnesium silicate, laponite, hectorite, and anhydrous silicic acid.

Also useful herein are carboxylic acid/carboxylate copolymers which are hydrophobically-modified cross-linked coplymers of carboxylic acid and alkyl carboxylate, and have an amphiphilic property. These carboxylic acid/carboxylate copolymers are obtained by copolymerizing 1) a carboxylic acid monomer such as acrylic acid, methacrylic acid, maleic acid, maleic anhydride, itaconic acid, fumaric acid, crotonic acid, or α-chloroacrylic acid, 2) a carboxylic ester having an alkyl chain of from 1 to about 30 carbons, and preferably 3) a crosslinking agent of the following formula: 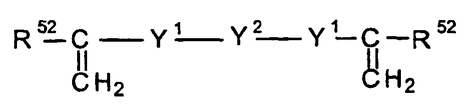 wherein R⁵² is a hydrogen or an alkyl group having from about 1 to about 30 carbons; Y¹, independently, is oxygen, CH₂O, COO, OCO, 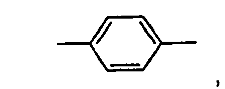 or 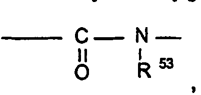 wherein R⁵³ is a hydrogen or an alkyl group having from about 1 to about 30 carbons; and Y² is selected from (CH₂)ₘ", (CH₂CH₂O)ₘ", or (CH₂CH₂CH₂O)ₘ" wherein m" is an integer of from 1 to about 30. It is believed that, because of the alkyl group contained in the copolymer, the carboxylic acid/carboxylate copolymers do not make the composition undesirably sticky.

Suitable carboxylic acid/carboxylate copolymers herein are acrylic acid/alkyl acrylate copolymers having the following formula: 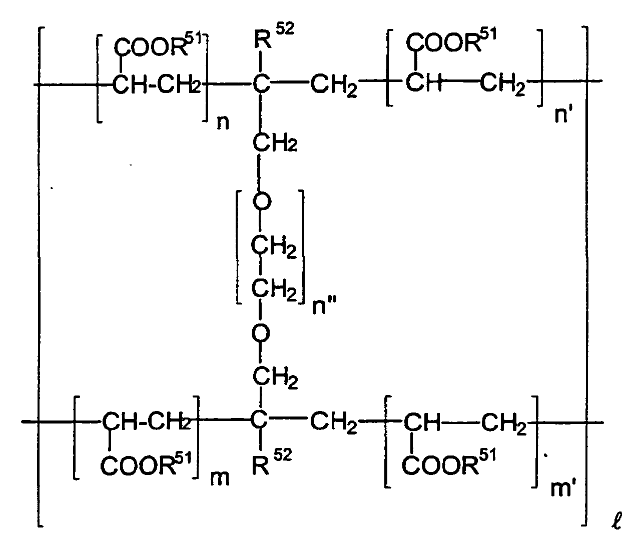 wherein R⁵¹, independently, is a hydrogen or an alkyl of 1 to 30 carbons wherein at least one of R⁵¹ is a hydrogen, R⁵² is as defined above, n, n', m and m' are integers in which n+n'+m+m' is from about 40 to about 100, n" is an integer of from 1 to about 30, and ℓ is defined so that the copolymer has a molecular weight of about 500,000 to about 3,000,000.

Commercially available carboxylic acid/carboxylate copolymers useful herein include: CTFA name Acrylates/C10-30 Alkyl Acrylate Crosspolymer having tradenames Pemulene TR-1, Pemulene TR-2, Carbopol 1342, Carbopol 1382, and Carbopol ETD 2020, all available from B. F. Goodrich Company.

Neutralizing agents may be included to neutralize the anionic thickening agents described hereinabove. Nonlimiting examples of such neutralizing agents include sodium hydroxide, potssium hydroxide, ammonium hydroxide, monethanolamine, diethanolamine, triethanolamine, diisopropanolamine, aminomethylpropanol, tromethamine, tetrahydroxypropyl ethylenediamine, and mixtures thereof.

Polyalkylene glycols having a molecular weight of more than about 1000 are useful herein. Useful are those having the following general formula: 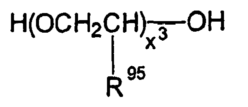 wherein R⁹⁵ is selected from the group consisting of H, methyl, and mixtures thereof. When R⁹⁵ is H, these materials are polymers of ethylene oxide, which are also known as polyethylene oxides, polyoxyethylenes, and polyethylene glycols. When R⁹⁵ is methyl, these materials are polymers of propylene oxide, which are also known as polypropylene oxides, polyoxypropylenes, and polypropylene glycols. When R⁹⁵ is methyl, it is also understood that various positional isomers of the resulting polymers can exist. In the above structure, x³ has an average value of from about 1500 to about 25,000, preferably from about 2500 to about 20,000, and more preferably from about 3500 to about 15,000. Other useful polymers include the polypropylene glycols and mixed polyethylene-polypropylene glycols, or polyoxyethylene-polyoxypropylene copolymer polymers,. Polyethylene glycol polymers useful herein are PEG-2M wherein R⁹⁵ equals H and X³ has an average value of about 2,000 (PEG-2M is also known as Polyox WSR^{®} N-10, which is available from Union Carbide and as PEG-2,000); PEG-5M wherein R⁹⁵ equals H and x³ has an average value of about 5,000 (PEG-5M is also known as Polyox WSR^{®} N-35 and Polyox WSR^{®} N-80, both available from Union Carbide and as PEG-5,000 and Polyethylene Glycol 300,000); PEG-7M wherein R⁹⁵ equals H and X³ has an average value of about 7,000 (PEG-7M is also known as Polyox WSR^{®} N-750 available from Union Carbide); PEG-9M wherein R⁹⁵ equals H and X³ has an average value of about 9,000 (PEG 9-M is also known as Polyox WSR^{®} N-3333 available from Union Carbide); and PEG-14 M wherein R⁹⁵ equals H and X³ has an average value of about 14,000 (PEG-14M is also known as Polyox WSR^{®} N-3000 available from Union Carbide).

Commercially available water-soluble thickening agents highly useful herein include xanthan gum with tradename KELTROL series available from Kelco, Carbomers with tradenames CARBOPOL 934, CARBOPOL 940, CARBOPOL 950, CARBOPOL 980, and CARBOPOL 981, all available from B. F. Goodrich Company, acrylates/steareth-20 methacrylate copolymer with tradename ACRYSOL 22 available from Rohm and Hass, nonoxynyl hydroxyethylcellulose with tradename AMERCELL POLYMER HM-1500 available from Amerchol, methylcellulose with tradename BENECEL, hydroxyethyl cellulose with tradename NATROSOL, hydroxypropyl cellulose with tradename KLUCEL, cetyl hydroxyethyl cellulose with tradename POLYSURF 67, all supplied by Herculus, ethylene oxide and/or propylene oxide based polymers with tradenames CARBOWAX PEGs, POLYOX WASRs, and UCON FLUIDS, all supplied by Amerchol.

### AQUEOUS CARRIER

The liquid compositions of the present invention comprise an aqueous carrier. The level and species of the carrier are selected according to the compatibility with other components, and other desired characteristic of the product.

Carriers useful in the present invention include water and water solutions of lower alkyl alcohols. Lower alkyl alcohols useful herein are monohydric alcohols having 1 to 6 carbons, more preferably ethanol and isopropanol.

Preferably, the aqueous carrier is substantially water. Deionized water is preferably used. Water from .natural sources including mineral cations can also be used, depending on the desired characteristic of the product.

The pH of the present composition is preferably from about 4 to about 8. When chronic whitening agents or other skin benefit agents are included in the liquid composition, the pH may be adjusted to that which provides optimum efficacy of the active skin benefit agents. Buffers and other pH adjusting agents can be included to achieve the desirable pH. Suitable pH adjusters herein include acetates, phosphates, citrates, triethanolamines and carbonates.

### CHRONIC WHITENING AGENT

The liquid composition may further contain a chronic whitening agent. The chronic whitening agent useful herein refers to active ingredients that not only alter the appearance of the skin, but further improve hyperpigmentation as compared to pre-treatment. By definition, chronic is referred to continued topical application of the composition over an extended period during the subject's lifetime, preferably for a period of at least about one week, more preferably for a period of at least about one month, even more preferably for at least about three months, even more preferably for at least about one year. Typically, applications would be on the order of about once per day over such extended periods, while application rates can vary from about once per week up to about three times per day or more. The chronic whitening agents may be included, by weight of the liquid composition, at a level preferably from about 0.001 % to about 10%, more preferably from about 0.1% to about 5%.

Useful chronic whitening agents useful herein include ascorbic acid compounds, vitamin B₃ compounds, azelaic acid, butyl hydroxy anisole, gallic acid and its derivatives, glycyrrhizinic acid, hydroquinoine, kojic acid, arbutin, mulberry extract, and mixtures thereof. Use of combinations of chronic whitening agents are believed to be advantageous in that they may provide whitening benefit through different mechanisms.

Ascorbic acid compounds are useful chronic whitening agents, and have the formula (I): 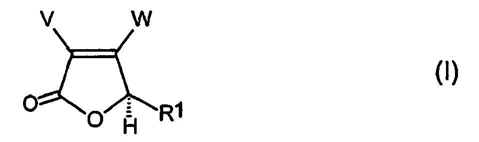 wherein V and W are independently -OH; R¹ is - CH(OH)-CH₂OH; and salts thereof.

Preferably, the ascorbic acid compound useful herein is an ascorbic acid salt or derivative thereof, such as the non-toxic alkali metal, alkaline earth metal and ammonium salts commonly known by those skilled in the art including, but not limited to, the sodium, potassium, lithium, calcium, magnesium, barium, ammonium and protamine salts which are prepared by methods well known in the art.

More preferably, the ascorbic acid salt useful herein is a metal ascorbate having the following formula (II): 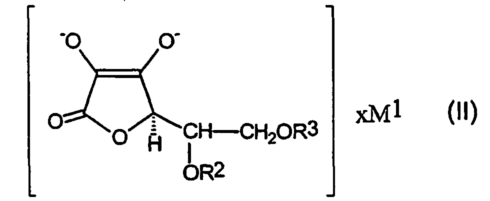 wherein R² and R³ are independently selected from hydrogen and linear or branched alkyl of 1 to about 8 carbons; M¹ is a metal; and x is an integer of from 1 to about 3. More preferably, R² and R³ are independently selected from hydrogen and linear or branched alkyl of 1 to about 3 carbons; M¹ is sodium, potassium, magnesium, or calcium.

Examples of other preferred ascorbic acid salts having formula (II) include monovalent metal salts (e.g., sodium ascorbate, potassium ascorbate), divalent metal salts (e.g., magnesium ascorbate, calcium ascorbate) and trivalent metal salts (e.g., aluminum ascorbate) of ascorbic acid.

Preferably, the ascorbic acid salt useful herein is a water soluble ascorbyl ester having the following formula (III): 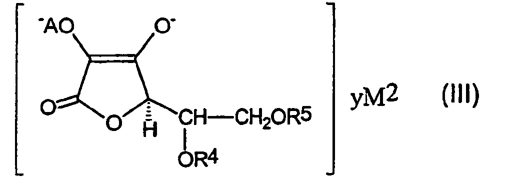 wherein A is sulfate or phosphate; R⁴ and R⁵ are independently selected from hydrogen and linear or branched alkyl of 1 to about 8 carbons; M² is a metal; and y is an integer of 1 to about 3. More preferably, R⁴ and R⁵ are independently selected from hydrogen and linear or branched alkyl of 1 to about 3 carbons; M² is sodium, potassium, magnesium, or calcium.

Another particularly preferred ascorbic acid compound is 2-o-α-D-glucopyranosyl-L-ascorbic acid, usually referred to as L-ascorbic acid 2-glucoside or ascorbyl glucoside, and its metal salts. Such compounds are available from Hayashibara.

Exemplary water soluble salt derivatives include, but are not limited to, L-ascorbic acid 2-glucoside, L-ascorbyl phosphate ester salts such as sodium L-ascorbyl phosphate, potassium L-ascorbyl phosphate, magnesium L-ascorbyl phosphate, calcium L-ascorbyl phosphate, aluminum L-ascorbyl phosphate. L-ascorbyl sulfate ester salts can also be used. Examples are sodium L-ascorbyl sulfate, potassium L-ascorbyl sulfate, magnesium L-ascorbyl sulfate, calcium L-ascorbyl sulfate and aluminum L-ascorbyl sulfate.

Vitamin B₃ compounds are useful as chronic whitening agents. The compounds are also known to provide skin appearance benefits such as regulating signs of skin aging such as wrinkles, lines, and pores. Vitamin B₃ compounds have the formula: 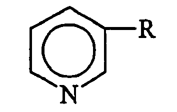 wherein R is -CONH₂ (*e.g.*, niacinamide), -COOH (e.g., nicotinic acid) or -CH₂OH (e.g., nicotinyl alcohol); derivatives thereof; and salts of any of the foregoing.

Exemplary derivatives of the foregoing vitamin B₃ compounds include nicotinic acid esters, including non-vasodilating esters of nicotinic acid, nicotinyl amino acids, nicotinyl alcohol esters of carboxylic acids, nicotinic acid N-oxide and niacinamide N-oxide.

Suitable esters of nicotinic acid include nicotinic acid esters of from 1 to about 22 carbons, preferably 1 to about 16 carbons, more preferably alcohols from about 1 to about 6 carbons. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (the majority of the general population would not experience a visible flushing response, although such compounds may cause vasodilation not visible to the naked eye, *i.e.,* the ester is non-rubifacient). Non-vasodilating esters of nicotinic acid include tocopherol nicotinate and inositol hexanicotinate; tocopherol nicotinate is preferred.

Other derivatives of the vitamin B₃ compound are derivatives of niacinamide resulting from substitution of one or more of the amide group hydrogens. Nonlimiting examples of derivatives of niacinamide useful herein include nicotinyl amino acids, derived; for example, from the reaction of an activated nicotinic acid compound (e.g., nicotinic acid azide or nicotinyl chloride) with an amino acid, and nicotinyl alcohol esters of organic carboxylic acids (e.g., 1 to about 18 carbons). Specific examples of such derivatives include nicotinuric acid (C₈H₈N₂O₃) and nicotinyl hydroxamic acid (C₆H₆N₂O₂), which have the following chemical structures:
nicotinuric acid: 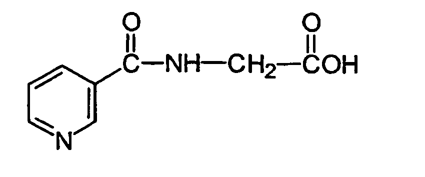
nicotinyl hydroxamic acid:

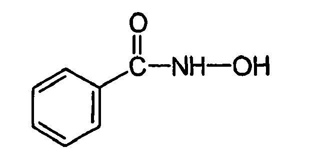

Exemplary nicotinyl alcohol esters include nicotinyl alcohol esters of the carboxylic acids salicylic acid, acetic acid, glycolic acid, palmitic acid and the like. Other non-limiting examples of vitamin B₃ compounds useful herein are 2-chloronicotinamide, 6-aminonicotinamide, 6-methylnicotinamide, n-methylnicotinamide, n,n-diethylnicotinamide, n-(hydroxymethyl)-nicotinamide, quinolinic acid imide, nicotinanilide, n-benzylnicotinamide, n-ethylnicotinamide, nifenazone, nicotinaldehyde, isonicotinic acid, methyl isonicotinic acid, thionicotinamide, nialamide, 1-(3-pyridylmethyl) urea, 2-mercaptonicotinic acid, nicomol, and niaprazine.

Nonlimiting examples of the above vitamin B₃ compounds are well known in the art and are commercially available from a number of sources, e.g., the Sigma Chemical Company (St. Louis, MO); ICN Biomedicals, Inc. (Irvin, CA) and Aldrich Chemical Company (Milwaukee, WI).

One or more vitamin B₃ compounds may be used herein. Preferred vitamin B₃ compounds are niacinamide and tocopherol nicotinate. Niacinamide is more preferred.

When used, salts, derivatives, and salt derivatives of niacinamide are preferably those having substantially the same efficacy as niacinamide in the methods of regulating skin condition described herein.

Salts of the vitamin B₃ compound are also useful herein. Nonlimiting examples of salts of the vitamin B₃ compound useful herein include organic or inorganic salts, such as inorganic salts with anionic inorganic species (e.g., chloride, bromide, iodide, carbonate, preferably chloride), and organic carboxylic acid salts (including mono-, di- and tri- C₁₋₁₈ carboxylic acid salts, e.g., acetate, salicylate, glycolate, lactate, malate, citrate, preferably monocarboxylic acid salts such as acetate). These and other salts of the vitamin B₃ compound can be readily prepared by the skilled artisan, for example, as described by W. Wenner, "The Reaction of L-Ascorbic and D-losascorbic Acid with Nicotinic Acid and Its Amide", J. Organic Chemistry, Vol. 14, 22-26 (1949). Wenner describes the synthesis of the ascorbic acid salt of niacinamide.

In a preferred embodiment, the ring nitrogen of the vitamin B₃ compound is substantially chemically free (e.g., unbound and/or unhindered), or after delivery to the skin becomes substantially chemically free ("chemically free" is hereinafter alternatively referred to as "uncomplexed"). More preferably, the vitamin B₃ compound is essentially uncomplexed. Therefore, if the composition contains the vitamin B₃ compound in a salt or otherwise complexed form, such complex is preferably substantially reversible, more preferably essentially reversible, upon delivery of the composition to the skin. For example, such complex should be substantially reversible at a pH of from about 5.0 to about 6.0. Such reversibility can be readily determined by one having ordinary skill in the art.

More preferably the vitamin B₃ compound is substantially uncomplexed in the composition prior to delivery to the skin. Exemplary approaches to minimizing or preventing the formation of undesirable complexes include omission of materials which form substantially irreversible or other complexes with the vitamin B₃ compound, pH adjustment, ionic strength adjustment, the use of surfactants, and formulating wherein the vitamin B₃ compound and materials which complex therewith are in different phases. Such approaches are well within the level of ordinary skill in the art.

Thus, in a preferred embodiment, the vitamin B₃ compound contains a limited amount of the salt form and is more preferably substantially free of salts of a vitamin B₃ compound. Preferably the vitamin B₃ compound contains less than about 50% of such salt, and is more preferably essentially free of the salt form. The vitamin B₃ compound in the compositions hereof having a pH of from about 4 to about 7 typically contain less than about 50% of the salt.

### WATER SOLUBLE HUMECTANT

The liquid composition of the present invention may further contain a water soluble humectant. Water soluble humectants are preferably included to provide moisturizing benefit to the skin. Further, water soluble humectants may help the dispersion of the water soluble thickening agents, and dissolving/dispersion of other components which are relatively difficult to process in an aqueous carrier. The water soluble humectants may be included, by weight of the liquid composition, at a level preferably from about 0.1 % to about 30%, more preferably from about 1% to about 20%, still preferably from about 5% to about 15%.

Water soluble humectants useful herein include polyhydric alcohols such as sorbitol, propylene glycol, butylene glycol, hexylene glycol, ethoxylated glucose, 1, 2-hexane diol, hexanetriol, dipropylene glycol, erythritol, trehalose, diglycerin, xylitol, maltitol, maltose, glucose, fructose, sodium chondroitin sulfate, sodium hyaluronate, sodium adenosin phosphate, sodium lactate, pyrrolidone carbonate, glucosamine, cyclodextrin, and mixtures thereof.

Water soluble humectants useful herein include water soluble alkoxylated nonionic polymers such as polyethylene glycols and polypropylene glycols having a molecular weight of up to about 1000 such as those with CTFA names PEG-200, PEG-400, PEG-600, PEG-1000, and mixtures thereof.

Commercially available humectants herein include: glycerin with tradenames STAR and SUPEROL available from The Procter & Gamble Company, CRODEROL GA7000 available from Croda Universal Ltd., PRECERIN series available from Unichema, and a same tradename as the chemical name available from NOF; propylene glycol with tradename LEXOL PG-865/855 available from Inolex, 1,2-PROPYLENE GLYCOL USP available from BASF; sorbitol with tradenames LIPONIC series available from Lipo, SORBO, ALEX, A-625, and A-641 available from ICI, and UNISWEET 70, UNISWEET CONC available from UPI; dipropylene glycol with the same tradename available from BASF; diglycerin with tradename DIGLYCEROL available from Solvay GmbH; xylitol with the same tradename available from Kyowa and Eizai; maltitol with tradename MALBIT available from Hayashibara, sodium chondroitin sulfate with the same tradename available from Freeman and Bioiberica, and with tradename ATOMERGIC SODIUM CHONDROITIN SULFATE available from Atomergic Chemetals; sodium hyaluronate with tradenames ACTIMOIST available from Active Organics, AVIAN SODIUM HYALURONATE series available from Intergen, HYALURONIC ACID Na available from Ichimaru Pharcos; sodium adenosin phophate with the same tradename available from Asahikasei, Kyowa, and Daiichi Seiyaku; sodium lactate with the same tradename available from Merck, Wako, and Showa Kako, cyclodextrin with tradenames CAVITRON available from American Maize, RHODOCAP series available from Rhone-Poulenc, and DEXPEARL available from Tomen; and polyethylene glycols with the tradename CARBOWAX series available from Union Carbide.

### ADDITIONAL COMPONENTS

Additional components may be included in the liquid compositions in view of the desired characteristics of the mask composition. Such additional components are included at a level that does not alter the function of the essential components of the mask composition of the present invention, typically no more than about 5% by weight of the liquid composition.

Additional components useful herein include additional skin benefit agents including, but are not limited to, anti-acne agents, anti-oxidants and radical scavengers, anti-inflammatory agents, antimicrobial agents, and skin texture improvement agents. Other Additional Components useful herein include surfactants, and preservatives.

### Anti-Acne Agents

Anti-acne actives can be effective in treating and preventing acne vulgais, a chronic disorder of the pilosebaceous follicles. Preferred anti-acne agents include salicylic acid, 4-methoxysalicylic acid, benzoyl peroxide, lactic acid, metronidazole, panthenol, retinoic acid and its derivaties, sulphur, triclosan, and mixtures thereof.

### Anti-Oxidants and Radical Scavengers

Anti-oxidants and radical scavengers are especially useful for providing protection against UV radiation which can cause increased scaling or texture changes in the stratum corneum and against other environmental agents which can cause skin damage.

Anti-oxidants and radical scavengers such as tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the tradename Trolox®), gallic acid and its alkyl esters, especially propyl gallate, uric acid and its salts and alkyl esters, sorbic acid and its salts, amines (*i.e.*, N,N-diethylhydroxylamine, aminoguanidine), sulfhydryl compounds (*i.e.,* glutathione), dihydroxy fumaric acid and its salts, lycine pidolate, arginine pilolate, nordihydroguaiaretic acid, bioflavonoids, lysine, methionine, proline, superoxide dismutase, silymarin, tea extracts, grape skin/seed extracts, melanin, and rosemary extracts may be used. Preferred anti-oxidants/radical scavengers are selected from tocopherol sorbate and other esters of tocopherol, more preferably tocopherol sorbate. For example, the use of tocopherol sorbate in topical compositions and applicable to the present invention is described in U.S. Patent 4,847,071, Bissett et al, issued July 11, 1989.

### Anti-Inflammatory Agents

Anti-inflammatory agents enhance the skin appearance benefits, by for example, contribution of uniformity and acceptable skin tone and/or color.

Preferably, the anti-inflammatory agent includes a steroidal anti-inflammatory agent and an non-steroidal anti-inflammatory agent. Preferred steroidal anti-inflammatory for use is hydrocortisone.

The variety of compounds encompassed by this group are well-known to those skilled in the art. For detailed disclosure of the chemical structure, synthesis, side effects, etc. of non-steroidal anti-inflammatory agents, reference may be had to standard texts, including Anti-inflammatory and Anti-Rheumatic Drugs, K. D. Rainsford, Vol. I-III, CRC Press, Boca Raton, (1985), and Anti-inflammatory Agents, Chemistry and Pharmacology, 1, R. A. Scherrer, et al., Academic Press, New York (1974).

So-called "natural" anti-inflammatory agents are also useful. Such agents may suitably be obtained as an extract by suitable physical and/or chemical isolation from natural sources (*i.e.,* plants, fungi, by-products of microorganisms). For example, alpha bisabolol, aloe vera, Manjistha (extracted from plants in the genus Rubia, particularly Rubia Cordifolia), and Guggal (extracted from plants in the genus Commiphora, particularly Commiphora Mukul), kola extract, chamomile, and sea whip extract, may be used.

Additional anti-inflammatory agents useful herein include compounds of the licorice (the plant genus/species Glycyrrhiza glabra) family, including glycyrrhetic acid, glycyrrhizic acid, and derivatives thereof (e.g., salts and esters). Suitable salts of the foregoing compounds include metal and ammonium salts. Suitable esters include C₂₋₂₄ saturated or unsaturated esters of the acids, preferably C₁₀₋₂₄, more preferably C₁₆₋₂₄.

### Antimicrobial Agent

As used, "antimicrobial agents" means a compound capable of destroying microbes, preventing the development of microbes or preventing the pathogenic action of microbes. Antimicrobal agents are useful, for example, in controlling acne. Preferred antimicrobial agents useful in the present invention are benzoyl peroxide, erythromycin, tetracycline, clindamycin, azelaic acid, sulfur resorcinol phenoxyethanol, and IRGASAN® DP 300 (Ciba Geigy Corp., U.S.A.). A safe and effective amount of an antimicrobial agent may be added to compositions of the present invention, preferably from about 0.001% to about 10%, more preferably from about 0.01% to about 5%, still more preferably from about 0.05% to about 2%.

### Skin Texture Improvement Agents

Skin texture treatment agents help repair and replenish the natural moisture barrier function of the epidermis, thereby providing skin benefits such as texture improvement.

Skin texture improvement agents useful herein are niacinamide, nicotinic acid and its esters, nicotinyl alcohol, panthenol, panthenyl ethyl ether, n-acetyl cysteine, n-acetyl-L-serine, phosphodiesterase inhibitors, trimethyl glycine, tocopheryl nicotinate, and vitamin B₃ and analogues or derivatives, and mixtures thereof. Panthenol is particularly preferred in that, when used in an amount of at least about 1%, it provides texture improvement benefits. Panthenol is commercially available, for example, by Roche.

### Surfactants

When water insoluble or hardly water soluble components are included in the liquid composition, a surfactant is needed to solubilize or disperse such components in the aqueous carrier.

Surfactants useful herein are cosmetically acceptable nonionic surfactants. Nonionic surfactants useful herein include condensation products of alkylene oxides which fatty acids, such as alkylene oxide esters of fatty acids, the condensation products of alkylene oxides with 2 moles of fatty acids, such as alkylene oxide diesters of fatty acids, the condensation products of alkylene oxides with fatty alcohols, examples of which include PEG 40 hydrogenated castor oil, steareth 2, isoceteth-20, and oleth-20. Other nonionic surfactants useful herein are the condensation products of alkylene oxides with both fatty acids and fatty alcohol, wherein the polyalkyene oxide portion is esterified on one end with a fatty acid and etherified on the other end with a fatty alcohol. Other nonionic surfactants useful herein are alkyl glucosides and alkyl polygycosides, polyhydroxy fatty acid amide surfactants, alkoxylated sugar esters and polyesters, and fatty acid amides.

Particularly useful nonionic surfactants herein are those selected from the group consisting of laureth-4, laureth-23, ceteareth-12, sucrose cocate, steareth-100, polysorbate-20, polysorbate-60, PEG-60 hydrogenated castor oil, isoceteth-20, oleth-20, PEG-100 stearate, and mixtures thereof. These nonionic surfactants are believed to act as emollients, and thus provide skin conditioning.

### Other components

In addition to the above described components, the composition of the present invention may further include preservatives and preservative enhancers such as water-soluble or dispersible preservatives including methyl paraben, propyl paraben, imidazolidinyl urea, Germall 115, methyl, ethyl, propyl and butyl esters of hydroxybenzoic acid, benzyl alcohol, EDTA, Bronopol (2-bromo-2-nitropropane-1,3-diol) and phenoxypropanol; ultraviolet light absorbers or scattering agents; sequestrants; anti-androgens; depilation agents; soluble or colloidally-soluble moisturizing agents such as hyaluronic acid and starch-grafted sodium polyacrylates such as SANWET® IM-1000, IM-1500 and IM-2500 available from Celanese Superabsorbent Materials, Portsmith, VA, USA and described in US Patent 4,076,663; proteins and polypeptides and derivatives thereof; organic hydroxy acids; drug astringents; external analgesics; film formers; anticaking agents; antifoaming agents; binders; coloring agents; perfumes, essential oils, and solubilizers thereof; natural extracts; and yeast fermented filtrates.

### METHOD OF PREPARATION

The mask composition of the present invention can be made by any method known to the artisan. Generally, the mask composition is made by placing the water-insoluble substrate in a housing, pouring in the liquid composition so that the water-insoluble substrate soaks the liquid composition, and sealed for delivery and/or storage. The mask composition can be housed in a package per unit or by multiple units. By "unit" what is meant is, for example, a single piece facial mask would make a unit by itself, while a two-piece facial mask would make a unit by one upper piece mask and one lower piece mask. Preferably, the mask composition is housed in individual packaging per unit. For single use packaging, the packaging is hermetically sealed and opened upon use. For multiple use packaging, the packaging is equipped by a means so that the packaging can be substantially hermetically sealed after opening.

The liquid composition can also be made by any method known to the artisan. The liquid composition can be suitably made by the following steps:
(a) dispersing the water-soluble thickening agent into water;
(b) adding the skin tone changing agent to the product of step (a); and
(c) mixing until homogeneous.

Water-soluble humectants are preferable components for the present liquid composition. When included in the liquid composition, such water-soluble humectants can be used to predisperse the water-soluble thickening agent prior to dispersing into water. The use of water-soluble humectant at step (a) may reduce the time required to disperse the water-soluble thickening agent.

### METHOD OF USE

The mask composition of the present invention is suitable for topical application on human body skin, particularly facial"skin. The use of the present composition provides acute skin tone changing benefit to the skin due to distribution and deposition of the skin tone changing agent on the skin.

Other benefits to the skin can be provided by application of the present mask composition in view of the specific benefit agents such as chronic whitening agents and skin benefit agents included in the liquid composition. The mask composition of the present invention is particularly advantageous in delivering the skin tone changing agent and other benefit agents in that the skin is exposed to an abundant amount of such agents over a lengthy period of time. Compared to when the liquid composition is applied to the skin without the use of the insoluble substrate, the use of the mask composition of the present invention, with the insoluble substrate as a delivery means over a lengthy period, is believed to provide better distribution and deposition of such agents, and better penetration of those agents which are percutaneously deliverable. Further, when an insoluble substrate having low air permeability is used, more effective penetration of the skin benefit agents into the skin is expected. The mask composition of the present invention is also believed to provide emotional benefits to the user upon use, such as refreshing feel, and relaxation feel.

In one preferred embodiment, the mask composition is used to treat the facial skin by the steps of:
(a) applying the mask composition to the majority of the area of the facial skin;
(b) allowing the mask composition to stand on the facial skin for a period of time no longer than until any portion of the mask composition is dried;
(c) removing the mask composition from the facial skin; and
(d) removing the remainder liquid composition left on the facial skin.
   The mask composition is soaked with an aqueous liquid composition, thus the mask fits to the facial skin by gently placing on the skin. For better fit and even distribution of the skin tone changing agent, the mask is pressed to the facial skin using finger tips.

By definition, "dried" refers to a state wherein water and other volatile components such as perfume, if included, evaporates from the water insoluble substrate, thereby leaving the substrate significantly less capable of delivering the skin tone changing agent to the skin. Thus, once a portion of the mask is dried, even distribution of the skin tone changing agent cannot be expected. Further, when dried, the mask composition provides an unpleasant stiff and tough feeling to the skin when applied.

Because the mask composition of the present invention is easily dried via exposure to regular atmospheric conditions, the mask composition must be housed in a hermetically sealed package during storage.

The period of time required until dried portions appear will depend on the atmosphere in which the use takes place, i.e. temperature, humidity, air circulation; and the structure and body temperature of the user. Typically, the mask composition should be designed so that no dried portions appear within a period of about 15 minutes when used in room temperature at a humidity of about 50%.

For effective delivery and distribution of chronic whitening agents and skin benefit agents, the mask composition in another preferred embodiment comprises a skin facing side and an occluded side, wherein the occluded side has an air permeability of less than about 5 mg/cm²/min. The mask composition of this embodiment is used by applying the mask composition to the majority of the area of the facial skin with the skin facing side of the mask composition touching the skin. The remaining steps for using the mask are the same as mentioned above. Such mask composition provides a humid environment for the face for a prolonged period of time. Further, when chronic whitening agents and/or skin benefit agents are included in the mask composition, better penetration of the chronic whitening agent and/or skin benefit agents are expected. When an insoluble substrate having low air permeability is used, the period of time by which the mask composition is dried can be prolonged, preferably from about 5 to about 45 minutes.

Accordingly, the present invention also relates to a method of chronically whitening the facial skin by use of the mask composition herein wherein the mask composition comprises a skin facing side and an occluded side, wherein the occluded side has an air permeability of less than about 5 mg/cm²/min, and further comprises a chronic whitening agent. The mask composition of this embodiment is used by applying the mask composition to the majority of the area of the facial skin with the skin facing side of the mask composition touching the skin. The remaining steps for using the mask are the same as mentioned above. By using a mask composition comprising an occluded side, significant increase of penetration of the chronic whitening agent is provided. Thus, application of such mask composition provides an effective method of chronically whitening the facial skin. For effective delivery and distribution of the chronic whitening agents and skin benefit agents, the mask composition is applied to the skin for preferably a period of at least about 5 minutes, preferably from about 5 to about 45 minutes.

### EXAMPLES

The following examples further describe and demonstrate embodiments within the scope of the present invention. The examples are given solely for the purpose of illustration and are not to be construed as limitations of the present invention. Where applicable, ingredients are identified by chemical or CTFA name, or otherwise defined below.

The mask compositions of Example 1 through 8 are made of about 2.5g of substrate RFP-90 available from Daiwabo, cut and shaped according to Fig. 1 and soaked with 30g each of the liquid compositions specified below. The mask compositions can also be made of 3.5g of cotton substrate instead of the substrate specified above.

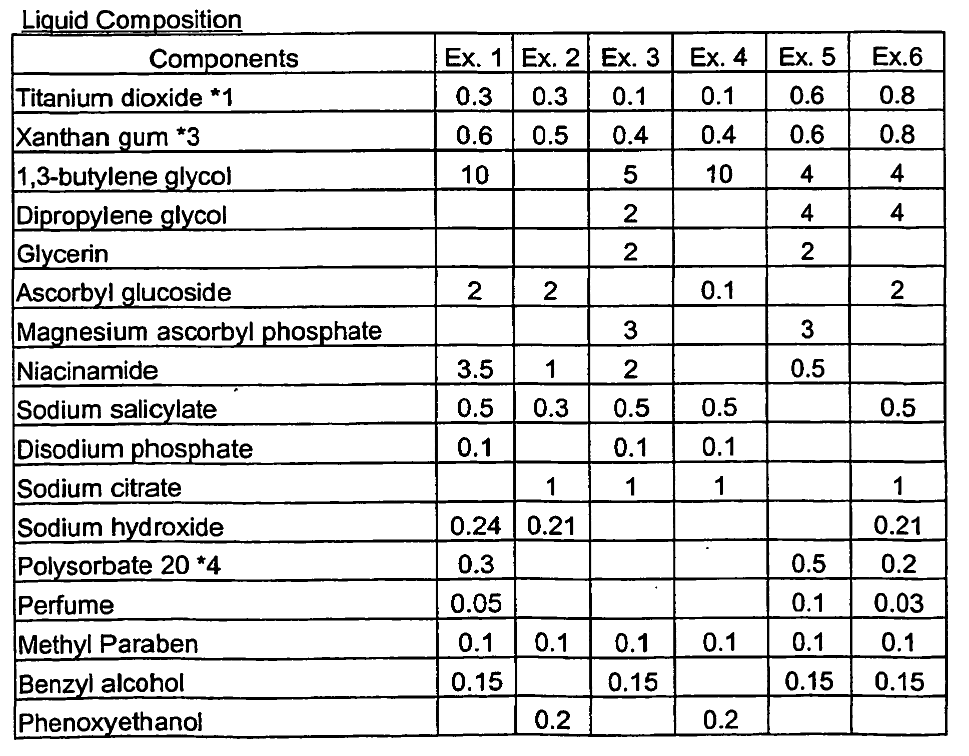

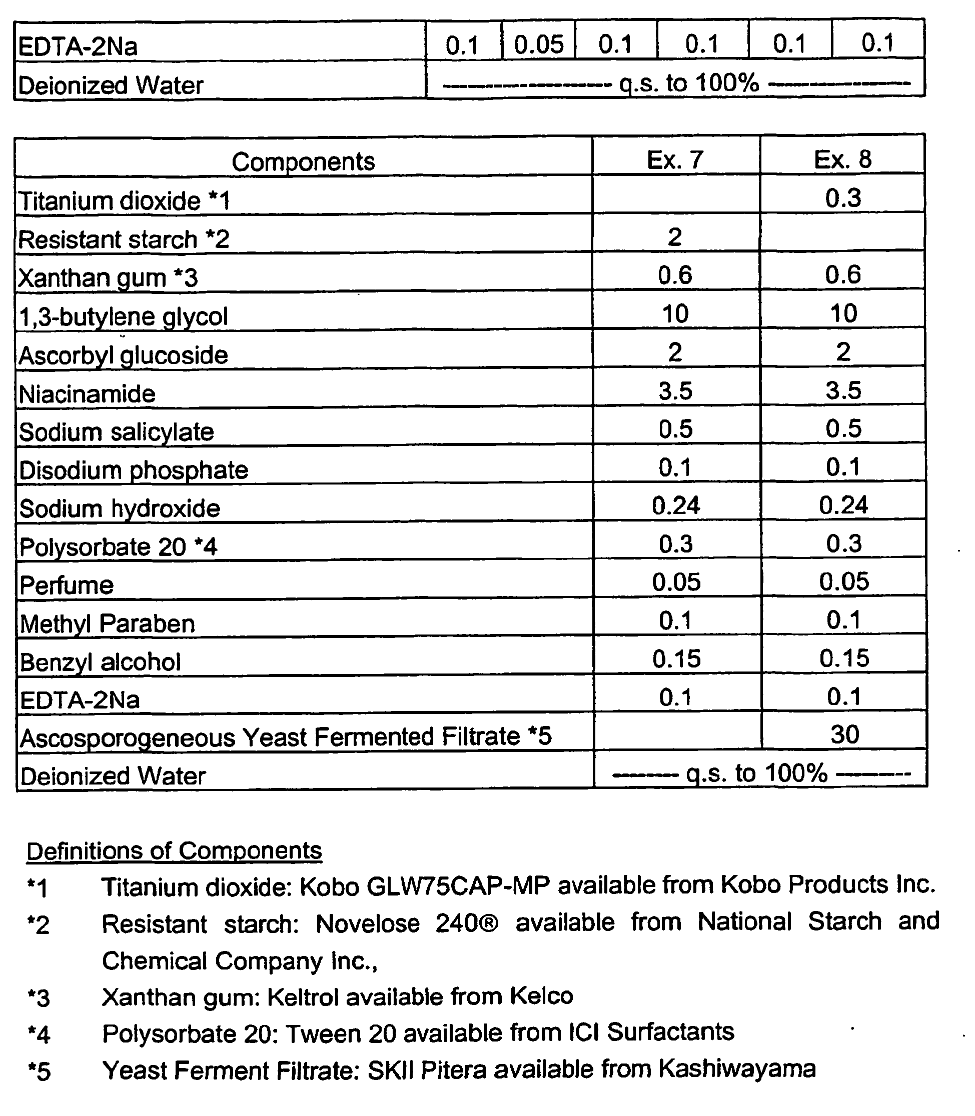

### Example 9

The mask composition of Example 9 is made of about 2.5g of substrate RFP-90 available from Daiwabo, cut and shaped according to Fig. 1, with the substrate laminated with a polyethylene sheet as shown in Fig. 2. The mask composition is then soaked with 30g of the liquid composition specified below. The mask composition can also be made of 3.5g of cotton substrate instead of the substrate specified above.

**Liquid Composition**

| Components | Ex. 9 |
|---|---|
| Treated Water | 83.543 |
| Xanthan gum *1 | 0.600 |
| Niacinamide | 3.500 |
| 1,3 Butylene Glycol | 10.000 |
| Sodium salicylate | 0.500 |
| Methylparaben | 0.100 |
| Benzyl alcohol | 0.150 |
| mulberry extract/water/butylene glycol | 0.500 |
| Disodium EDTA | 0.100 |
| Disodium phosphate 2. H₂O | 0.150 |
| Sodium phosphate H₂O | 0.115 |
| Polysorbate 20*3 | 0.300 |
| Perfume | 0.020 |
| Titanium dioxide *4 | 0.272 |
| Magnesium ascorbyl phosphate | 0.140 |
| Natural extracts *5 | 0.010 |
| Sodium sulfite anhydrous | 0.100 |
| Total | 100.000 |

### Definitions of Components

*1 Xanthan gum : Keltrol available from Kelco
*2 mulberry extract/water/butylenes glycol: mulberry extract BG available from Kobo.
*3 Polysorbate 20: Tween 20 available from ICI Surfactants
*4 Titanium dioxide: Kobo GLW75CAP-MP available from Kobo Products Inc.
*5 Natural extracts: Arnica Mountan Flower Extract, Hypericum Perforatum Extract, Ivy (Hedera Helix) extract, witchhazel (Hamamelis Virginiana) extract, grape (vitis vinifera) Leaf extract, horse chestnut (aesculus hippocastanum) extract, butylenes glycol, and water: Phytlene available from Kaneda.

### Method of Preparation

The compositions above described can be made by any method known to the artisan. The compositions are suitably made as follows:
(1) Any oily component such as perfume, if present, is pre-dissolved into surfactant and a small amount of water.
(2) The water-soluble thickening agent is pre-dispersed into water and/or water-soluble humectant, if present.
(3) All remaining components except the skin tone changing agent is dissolved in water and/or water soluble humectant, and the pH is adjusted, as necessary, by a pH adjusting agent. Heat may be added as necessary. When present, relatively oily components such as preservatives can be pre-dissolved in water-soluble humectant.
(4) The products of steps (1) and (3) are mixed until homogeneous.
(5) The skin tone changing agent is added to the product of step (4) and mixed until homogeneous, using a high speed mixer as necessary.
(6) The products of steps (2) and (5) are mixed until homogeneous, using a high speed mixer as necessary.
(7) The water-insoluble substrate is folded and placed in an aluminium pouch per one unit.
(8) The liquid composition thus obtained at step (6) is poured into the aluminum pouch containing the water-insoluble substrate, and hermetically sealed.

The embodiments of the present invention disclosed and represented above have many advantages. When applied to the face using finger tips for good fit and left for about 15 minutes, it provides an acute whitening benefit, and alleviates uneven skin tone, skin dullness, and skin dryness. When Example 9 is applied on the face, enhanced penetration of chronic whitening agents results.

## Claims

1. A mask composition comprising:
(1) a water insoluble non woven substrate; and
(2) a liquid composition comprising:
(a) a skin tone changing agent selected from the group consisting of skin tone changing pigments, reflective particulate material, and mixtures thereof, wherein the skin tone changing agent has a particle size of at least 100nm;
(b) a water-soluble thickening agent which provides the liquid composition a viscosity of from 1000 mPa s to 600,000 mPa s as measured by a Brookfield Digital Viscometer, Model DV-II+ Version 3.2 according to the operating instructions set forth in Manual No M/92-161-H895;
(c) an aqueous carrier.

2. The mask composition of Claim 1 wherein the skin tone changing agent is from 0.001 % to 3% of reflective particulate material.

3. The mask composition of Claim 2, wherein the reflective particulate material has an average particle size of from 100nm to 10µm.

4. The mask composition of Claim 3, wherein the reflective particulate material is a metallic oxide having a particle size of from 100nm to 1000nm.

5. The mask composition of Claim 3, wherein the reflective particulate material is a resistant starch.

6. The mask composition of Claim 1 wherein the thickening agent contains xanthan gum.

7. The mask composition of Claim 1 further comprising a chronic whitening agent.

8. The mask composition of Claim 7 wherein the chronic whitening agent contains at least an ascorbic acid derivative and a vitamin B₃ derivative.

9. The mask composition of Claim 1 further comprising a water-soluble humectant

10. The mask composition of Claim 1 further comprising a perfume.

11. The mask composition of Claim 1 wherein the water-insoluble substrate is at least partially made by hydrophilic materials selected from cotton, pulp, rayon, and mixtures thereof.

12. The mask composition of Claim 11 wherein the water-insoluble substrate comprises a skin facing side and an occluded side, the occluded side having an air permeability of less than 5 mg/cm²/min.

13. The mask composition of Claim 12 wherein the occluded side of the substrate is made of hydrophobic material.

14. The mask composition of Claim 12 wherein the skin facing side is made of hydrophilic material.

15. The mask composition of Claim 12 wherein the occluded side of the substrate is laminated with a polymeric film.

16. The mask composition of Claim 12 wherein the liquid composition further contains a chronic whitening agent.

17. The mask composition of Claim 11 wherein the water-insoluble substrate is so configured to cover the majority of the area of facial skin.

18. The mask composition of Claim 11 wherein the water-insoluble substrate has a thickness of from 100µm to 1 cm.

19. The mask composition of Claim 11 comprising a water-insoluble substrate configured as a single-piece whole facial mask.

20. The mask composition of Claim 11 comprising a first piece and a second piece of the water-insoluble substrate, the first piece being so configured to cover the upper half of the facial skin, and the second piece being so configured to cover the lower half of the facial skin.

21. A method of treating the facial skin comprising the steps of:
(a) applying the mask composition of Claim 1 to the majority of the area of the facial skin;
(b) allowing the mask composition to stand on the facial skin for a period of time no longer than until any portion of the mask composition is dried;
(c) removing the mask composition from the facial skin; and
(d) removing the remainder liquid composition left on the facial skin.

22. The method of claim 21 wherein, at step (b), the period of time is from about 5 to about 45 minutes.

23. The method of claim 21 wherein, at step (a), the mask is applied and fit on the facial skin by pressing on the mask to the facial skin using finger tips.

## Patentansprüche

1. Maskenzusammensetzung, die Folgendes umfasst:
(1) ein wasserunlösliches Vliessubstrat; und
(2) eine flüssige Zusammensetzung, die Folgendes umfasst:
(a) ein hauttonänderndes Mittel ausgewählt aus der Gruppe, bestehend aus hauttonändernden Pigmenten, reflektierendem Teilchenmaterial und Mischungen davon, wobei das hauttonändernde Mittel eine Teilchengröße von mindestens 100 nm aufweist;
(b) ein wasserlösliches Verdickungsmittel, das der flüssigen Zusammensetzung eine Viskosität von 1000 mPa.s bis 600.000 mPa.s verleiht; entsprechend einer Messung durch ein digitales Brookfield-Viskosimeter, Modell DV-II + Version 3.2 gemäß den im Handbuch Nr. M/92-161-M895 ausgeführten Bedienungsanweisungen
(c) einen wässrigen Träger.

2. Maskenzusammensetzung nach Anspruch 1, wobei das hauttonändernde Mittel von 0,001 % bis 3 % ein reflektierendes Teilchenmaterial ist.

3. Maskenzusammensetzung nach Anspruch 2, wobei das reflektierende Teilchenmaterial eine durchschnittliche Teilchengröße von 100 nm bis 10 µm auf weist.

4. Maskenzusammensetzung nach Anspruch 3, wobei das reflektierende Teilchenmaterial ein Metalloxid mit einer Teilchengröße von 100 nm bis 1000 nm ist.

5. Maskenzusammensetzung nach Anspruch 3, wobei das reflektierende Teilchenmaterial eine resistente Stärke ist.

6. Maskenzusammensetzung nach Anspruch 1, wobei das Verdickungsmittel Xanthangummi enthält.

7. Maskenzusammensetzung nach Anspruch 1, die ferner einen chronischen Weißmacher umfasst.

8. Maskenzusammensetzung nach Anspruch 7, wobei der chronische Weißmacher zumindest ein Ascorbinsäurederivat und ein Vitamin-B₃-Derivat enthält.

9. Maskenzusammensetzung nach Anspruch 1, die ferner ein wasserlösliches Feuchthaltemittel umfasst.

10. Maskenzusammensetzung nach Anspruch 1, die ferner einen Duftstoff umfasst.

11. Maskenzusammensetzung nach Anspruch 1, wobei das wasserunlösliche Substrat mindestens teilweise aus hydrophilen Materialien, ausgewählt aus Baumwolle, Zellstoff, Rayon und Mischungen davon, hergestellt wird.

12. Maskenzusammensetzung nach Anspruch 11, wobei das wasserunlösliche Substrat eine hautseitige Seite und eine okkludierte Seite umfasst, wobei die okkludierte Seite eine Luftdurchlässigkeit von unter 5 mg/cm²/min aufweist.

13. Maskenzusammensetzung nach Anspruch 12, wobei die okkludierte Seite des Substrats aus hydrophobem Material hergestellt ist.

14. Maskenzusammensetzung nach Anspruch 12, wobei die hautseitige Seite aus hydrophilem Material hergestellt ist.

15. Maskenzusammensetzung nach Anspruch 12, wobei die okkludierte Seite des Substrats mit einer Polymerfolie laminiert ist.

16. Maskenzusammensetzung nach Anspruch 12, wobei die flüssige Zusammensetzung ferner einen chronischen Weißmacher enthält.

17. Maskenzusammensetzung nach Anspruch 11, wobei das wasserunlösliche Substrat so ausgelegt ist, dass es den Großteil der Fläche der Gesichtshaut abdeckt.

18. Maskenzusammensetzung nach Anspruch 11, wobei das wasserunlösliche Substrat eine Dicke von 100 µm bis 1 cm aufweist.

19. Maskenzusammensetzung nach Anspruch 11, die ein wasserunlösliches Substrat umfasst, das als einstückige ganze Gesichtsmaske ausgelegt ist.

20. Maskenzusammensetzung nach Anspruch 11, die ein erstes Stück und ein zweites Stück des wasserunlöslichen Substrats umfasst, wobei das erste Stück so ausgelegt ist, dass es die obere Hälfte der Gesichtshaut abdeckt, und das zweite Stück so ausgelegt ist, dass es die untere Hälfte der Gesichtshaut abdeckt.

21. Verfahren zum Behandeln der Gesichtshaut, das folgende Schritte umfasst:
(a) Aufbringen der Maskenzusammensetzung nach Anspruch 1 auf den Großteil der Fläche der Gesichtshaut;
(b) Verweilenlassen der Maskenzusammensetzung auf der Gesichtshaut über einen Zeitraum, der nicht länger dauert, als bis ein beliebiger Teil der Maskenzusammensetzung getrocknet ist;
(c) Entfernen der Maskenzusammensetzung von der Gesichtshaut; und
(d) Entfernen der restlichen flüssigen Zusammensetzung, die auf der Gesichtshaut verblieben ist.

22. Verfahren nach Anspruch 21, wobei bei Schritt (b) der Zeitraum von etwa 5 bis etwa 45 Minuten beträgt.

23. Verfahren nach Anspruch 21, wobei bei Schritt (a) die Maske auf die Gesichtshaut aufgebracht und angepasst wird, indem die Maske mithilfe der Fingerspitzen auf die Gesichtshaut gedrückt wird.

## Revendications

1. Composition de masque comprenant :
(1) un substrat non tissé insoluble dans l'eau ; et
(2) une composition liquide comprenant :
(a) un agent de modification de teint choisi dans le groupe constitué de pigments de modification du teint, une matière particulaire réfléchissante, et leurs mélanges, dans laquelle l'agent de modification du teint a une dimension des particules d'au moins 100 nm ;
(b) un agent épaississant hydrosoluble qui confère à la composition liquide une viscosité comprise entre 1000 mPa.s et 600 000 mPa.s ; telle que mesurée par un viscosimètre numérique Brookfield, Modèle DV-II + Version 3.2 selon le mode d'emploi représenté dans le Manuel No. M/92-161-M895
(c) un véhicule aqueux.

2. Composition de masque selon la revendication 1, dans laquelle l'agent de modification du teint est compris entre 0,001 % et 3 % de la matière particulaire réfléchissante.

3. Composition de masque selon la revendication 2, dans laquelle la matière particulaire réfléchissante a une taille de particule moyenne comprise entre 100 nm et 10 µm.

4. Composition de masque selon la revendication 3, dans laquelle la matière particulaire réfléchissante est un oxyde métallique ayant une dimension des particules comprise entre 100 nm et 1000 nm.

5. Composition de masque selon la revendication 3, dans laquelle la matière particulaire réfléchissante est un amidon résistant.

6. Composition de masque selon la revendication 1, dans laquelle l'agent épaississant contient de la gomme de xanthane.

7. Composition de masque selon la revendication 1, comprenant en outre un agent procurant de la blancheur chronique.

8. Composition de masque selon la revendication 7, dans laquelle l'agent procurant de la blancheur chronique contient au moins un dérivé d'acide ascorbique et un dérivé de vitamine B₃.

9. Composition de masque selon la revendication 1, comprenant en outre unhumidifiant hydrosoluble.

10. Composition de masque selon la revendication 1, comprenant en outre un parfum.

11. Composition de masque selon la revendication 1, dans laquelle le substrat insoluble dans l'eau est au moins partiellement composé de matières hydrophiles choisies parmi le coton, la pâte, la rayonne, et leurs mélanges.

12. Composition de masque selon la revendication 11, dans laquelle le substrat insoluble dans l'eau comprend un côté faisant face à la peau et un côté occlus, le côté occlus ayant une perméabilité à l'air inférieure à 5 mg/cm²/min.

13. Composition de masque selon la revendication 12, dans laquelle le côté occlus du substrat est constitué d'un matériau hydrophobe.

14. Composition de masque selon la revendication 12, dans laquelle le côté faisant face à la peau est constitué d'un matériau hydrophile.

15. Composition de masque selon la revendication 12, dans laquelle le côté occlus du substrat est stratifié avec un film polymère.

16. Composition de masque selon la revendication 12, dans laquelle la composition liquide contient en outre un agent procurant de la blancheur chronique.

17. Composition de masque selon la revendication 11, dans laquelle le substrat insoluble dans l'eau est configuré de façon à protéger la majorité de la zone de la peau du visage.

18. Composition de masque selon la revendication 11, dans laquelle le substrat insoluble dans l'eau a une épaisseur comprise entre 100 µm et 1 cm.

19. Composition de masque selon la revendication 11, comprenant un substrat insoluble dans l'eau configuré comme un masque pour le visage complet en une seule pièce.

20. Composition de masque selon la revendication 11, comprenant une première pièce et une deuxième pièce du substrat insoluble dans l'eau, la première pièce étant configurée de façon à protéger la moitié supérieure de la peau du visage, et la deuxième pièce étant configurée pour protéger la moitié inférieure de la peau du visage.

21. Procédé de traitement de la peau du visage comprenant les étapes consistant à :
(a) appliquer la composition de masque selon la revendication 1 sur la majorité de la zone de la peau du visage ;
(b) permettre à la composition de masque de rester sur la peau du visage pendant une période de temps pas plus longue que jusqu'à ce que n'importe quelle partie de la composition de masque soit séchée ;
(c) enlever la composition de masque de la peau du visage ; et
(d) enlever la composition liquide restante laissée sur la peau du visage.

22. Procédé selon la revendication 21 dans lequel, à l'étape (b), la période de temps est comprise entre environ 5 et environ 45 minutes.

23. Procédé selon la revendication 21, dans lequel, à l'étape (a), le masque est appliqué et ajusté sur la peau du visage en appuyant sur le masque pour l'appliquer contre la peau du visage du bout des doigts.
